# EUROPÄISCHE PATENTANMELDUNG

(11) **EP 0 617 993 A1**
(43) Veröffentlichungstag der Anmeldung: **05.10.1994**
(21) Anmeldenummer: 93105152.8
(22) Anmeldetag: 29.03.1993
(51) Int. Cl.: B01D 39/20, B01D 71/02, C04B 38/00, A61L 2/02, C02F 1/44

(54) **Mikrofilterscheibe und Verfahren zu ihrer Herstellung**

(71) Anmelder: LUKOPAT AG, CH-8750 Glarus (CH)
(72) Erfinder: Die Erfindernennung liegt noch nicht vor
(74) Vertreter: Bardehle, Heinz, Dipl.-Ing.

(57) **Zusammenfassung**

Die vorliegende Erfindung betrifft eine Mikrofilterscheibe auf Kieselgurbasis, insbesondere zur vollständigen Entkeimung von Wasser, wobei die Mikrofilterscheibe Anteile von mindestens zwei silikatisch gebundenen Kieselgurarten enthält, von denen mindestens eine vorkalziniert und später gebrannt ist. Die Mikrofilterscheibe weist bevorzugt Porengrößen in der Größenordnung von 0,1 bis 0,3 µ auf, wodurch Bakterien sicher mechanisch zurückgehalten werden können. Ein Verfahren unter der Verwendung von drei unterschiedlichen Kieselgurarten und Kaolin in Verbindung mit einer vorsichtigen Trocknung und anschließendem Brennen macht die Herstellung von Scheiben mit Durchmesser größer als 35 cm und einer Dicke von 1 bis 1,5 cm möglich. Solche Scheiben können durch rotierende Bürsten leicht mechanisch abtragend gesäubert werden, so daß sie den einfachen Aufbau einer selbstreinigenden Filtereinrichtung ermöglichen. So aufgebaute Filteranordnungen sind wartungsarm und aufgrund der Einfachheit der Filterelemente auch durch Nicht-Fachleute zu warten.

## Beschreibung

Die vorliegende Erfindung betrifft eine Mikrofilterscheibe, insbesondere zur Entkeimung von Wasser, und ein Verfahren zu ihrer Herstellung.

Bei der mechanischen Entkeimung von Wasser durch ein Mikrofilter kommt es darauf an, daß die Porengröße der Filtervorrichtung so klein ist, daß Bakterien mit einer typischen Größe von 0,4 bis 0,5 µ sicher zurückgehalten werden. Keramiken oder andere Materialien mit geeigneten Porengrößen sind bisher nicht in beliebigen Formen herstellbar, so daß großflächige Filter mit guter Filterwirkung nur mit großen Aufwand herzustellen und zu warten waren. Bisher verwendete großflächige Filterkerzen aus Keramik mit geeigneter Porengröße lassen sich nur schwer reinigen, wobei solche Filter im allgemeinen regelmäßig geöffnet und von Hand gesäubert werden müssen.

In der nicht vorveröffentlichten europäischen Patentanmeldung 92 810 843, auf die hier ausdrücklich Bezug genommen wird, ist eine selbstreinigende Filtereinrichtung beschrieben, die flache keramische Filterelemente enthält, wobei Reinigungseinheiten vorgesehen sind, die relativ zu den Filterflächen bewegbar und an diese unter Abarbeitung von Filtermaterial unter Druck anlegbar sind. Solche Filter lassen sich durch bewegliche und von außen angetriebene Reinigungsbürsten in zusammengebautem Zustand regelmäßig reinigen, wodurch sich die Wartung wesentlich vereinfacht.

Aufgabe der vorliegenden Erfindung ist es, eine großflächige flache Mikrofilterscheibe zur Verfügung zu stellen, welche z. B. in einer selbstreinigenden Filtereinrichtung eingesetzt werden kann. Außerdem soll ein geeignetes Verfahren zur Herstellung einer solchen Mikrofilterscheibe angegeben werden.

Zur Lösung dieser Aufgabe ist eine Mikrofilterscheibe geeignet, welche auf Kieselgurbasis hergestellt ist und mindestens zwei silikatisch gebundene Kieselgurarten enthält, von denen mindenstens eine vorkalzimiert und später gebrannt ist und wobei die Mikrofilterscheibe Porengrößen in der Größenordnung von 0,05 bis 0,5 µ, vorzugsweise 0,1 bis 0,3 µ aufweist. Es hat sich gezeigt, daß ein Anteil an vorkalziniertem Kieselgur bei der Herstellung die Stabilität der Mikrofilterscheibe gewährleistet und ein Gerüst für die Anordnung mindestens eines weiteren Anteils Kieselgur einer anderen Art bildet, wodurch sich erst eine stabile Form mit der gewünschten Porengröße ergibt. Insbesondere ist so eine monolithische runde Mikrofilterscheibe mit einem Durchmesser von mindestens 25 cm, vorzugsweise mindestens 35 cm, herstellbar. Eine solche Scheibe kann eine Dicke von 0,5 bis 2 cm, vorzugsweise etwa 1 bis 1,5 cm, aufweisen. Eine gute Filterwirkung bei gleichzeitiger Bruchfestigkeit ergibt sich etwa bis zu einer Dicke von 0,5 cm. Eine typische Durchlässigkeit einer erfindungsgemäßen Scheibe beträgt 0,5 bis 0,7 l/(min x dm²), vorzugsweise etwa 0,6.

Bevorzugt weist die Mikrofilterscheibe eine zentrale, vorzugsweise runde Öffnung auf, mit einem Durchmesser von 3 bis 15 cm, vorzugsweise 6 bis 10 cm. Mittels dieser Öffnung können solche Mikrofilterscheiben auf einer zentralen Säule gestapelt werden, wobei die Säule drehbar sein kann oder eine drehbare Achse enthalten kann, um eine Relativbewegung zwischen Mikrofilterscheiben und Reinigungsvorrichtungen zu ermöglichen.

Um solche Mikrofilterscheiben dicht in eine Filtervorrichtung einbauen zu können, müssen die Außen- und Innenränder der Mikrofilterscheibe maßhaltig geschnitten sein, was vorzugsweise mit einem Hochdruckwasserstrahlschneider erreichbar ist. Die Ränder werden bevorzugt mit einer Dichtung versehen, welche die Scheibe zumindest an einer Seite um 1 bis 8 mm, vorzugsweise 3 bis 5 mm, umgreift. Auf diese Weise kann die Scheibe sowohl seitlich wie gegenüber einer Unterlage dichtend montiert werden. Die Dichtungen an den Innen- und Außenseiten sollten etwa 1 - 3 mm dick sein, um geringfügige Größenänderungen der Scheibe ausgleichen zu können.

Um von Zeit zu Zeit eine automatische Reinigung der Oberfläche der Filterscheibe zu ermöglichen, weist diese bevorzugt eine offenporige Oberfläche auf, welche so weich ist, daß sie mittels einer Reinigungsbürste unter Erzielung eines Abriebes bearbeitet werden kann. Eine solche Reinigungsbürste kann beispielsweise Kunststoffborsten, insbesondere aus Nylon, oder Edelmetallborsten aufweisen, wobei zu einer Oberflächenreinigung in wenigen Arbeitsgängen ein Abrieb von 0,1 bis 5 µ erzielt werden soll. Dieser Abrieb soll nach ein bis zwanzig Relativbewegungen zwischen Oberfläche und Reinigungsbürste erreicht sein. Ein solcher Abrieb kann bei einem Spülvorgang anschließend ausgespült werden und gelangt nicht durch den Filter.

Zur Abtötung von eindringenden Keimen und zur Verhinderung eines Durchwachsens von auf der Oberfläche zurückgehaltenen Bakterien durch die Mikrofilterscheibe enthält diese bevorzugt nicht auswaschbare Anteile an Silbersalzen.

Eine Scheibe mit besonders großer Stabilität und guter Filterwirkung läßt sich durch die Verwendung von mindestens drei verschiedenen Kieselgurkomponenten und einem Anteil Kaolin erreichen. Dabei ist eine der Kieselgurkomponenten vorkalziniert und mindestens eine der Kieselgurkomponenten enthält besonders viele kleine Partikel, insbesondere etwa 15 % Partikel mit einer Größe unter 2 µ.

Bei einer bevorzugten Ausführungsform enthält die Mikrofilterscheibe 6 bis 12 Gewichtsprozent, vorzugsweise etwa 9 Gewichtsprozent, eines vorkalzinierten und gemeinsam mit den übrigen Bestandteilen nochmals gebrannten Kieselgurs, 25 bis 30 Gewichtsprozent Kaolin und als Rest mindestens eine weitere Sorte Kieselgur sowie gegebenenfalls Silbersalze und andere geringe herstellungsbedingte Bestandteile. Die Gewichtsprozente beziehen sich dabei auf die gebrannte trockene Scheibe.

Die Herstellung einer solchen Mikrofilterscheibe wird durch ein Verfahren mit folgenden Schritten gelöst:
a. Es werden gemischt: Mindestens 6 bis 12 Gewichtsteile, vorzugsweise etwa 9 Gewichtsteile, einer schon kalzinierten Sorte Kieselgur, mit 25 bis 35 Gewichtsteilen, vorzugsweise etwa 30 Gewichtsteilen, Kaolin und 53 bis 69 Gewichtsteile, vorzugsweise etwa 61 Gewichtsteile, mindestens einer nicht oder nur wenig kalzinierten Sorte Kieselgur;
b. Die Mischung wird angerührt in etwa 200 bis 250 Gewichtsteilen, vorzugsweise etwa 225 Gewichtsteilen, Wasser, unter Zugabe von 0,2 bis 0,35 Gewichtsteilen, vorzugsweise 0,27, mindestens eines Verflüssigungsmittels zur Herabsetzung der Oberflächenspannung und von 0,4 bis 0,8 Gewichtsteilen, vorzugsweise 0,5 bis 0,7, eines organischen Bindemittels;
c. Die Mischung wird mindestens so lange gerührt, bis sich eine deutliche Viskositätsänderung einstellt, vorzugsweise etwa 12 bis 15 Stunden;
d. Die fertig gerührte Mischung wird in eine Form aus Feuchtigkeit aufnehmenden Material, vorzugsweise Gips, gegossen;
e. Die Mischung wird langsam getrocknet, vorzugsweise über einen Zeitraum von 8 bis 25 Tagen, insbesondere 10 bis 20 Tagen;
f. Die getrocknete Mischung wird gebrannt, vorzugsweise bei einer Maximaltemperatur von 1000 bis 1050 ° C, insbesondere etwa 1035 ° C.

Wie anhand der Ausführungsbeispiele noch näher erläutert wird, bewirkt das Verflüssigungsmittel in Verbindung mit dem langen Rühren eine sehr gleichmäßige Verteilung der feinen Partikel der unterschiedlichen Kieselgursorten und des Kaolins. Das organische Bindemittel sorgt für einen Zusammenhalt der noch nicht gebrannten Scheibe beim Trocknen in und außerhalb der Form, während der schon kalzinierte Anteil Kieselgur ein stabiles Gerüst bildet und damit die Formstabilität (Grünfestigkeit) der noch nicht gebrannten Scheibe sichert. Durch den Anteil an kalzinierten Kieselgur stabilisiert und durch das organische Bindemittel zusammengehalten ordnen sich die übrigen Bestandteile sehr homogen so an, daß die gewünschte Porosität entsteht. Auf diese Weise ist es möglich, monolithische Scheiben mit bisher nicht erreichten Durchmessern und der gewünschten Porosität rißfrei herzustellen.

In einer bevorzugten Ausführungsform werden folgende Gewichtsanteile der Hauptbestandteile verwendet:
- etwa 40,72 eines ersten unkalzinierten oder wenig kalzinierten Kieselgurs,
- etwa 9,05 eines zweiten kalzinierten Kieselgurs,
- etwa 20,83 eines dritten unkalzinierten oder wenig kalzinierten Kieselgurs, und
- etwa 29,41 Kaolin.
Dabei werden Kieselgurarten unterschiedlicher Verteilung der Größe der Partikel eingesetzt, um insgesamt die gewünschte Mikroporosität in der Größenordnung von 0,05 bis 0,5 µ, vorzugsweise 0,1 bis 0,3 µ, zu erreichen.

Um zu verhindern, daß später an der Oberfläche zurückgehaltene Bakterien durch die Keramik hindurchwachsen können, werden der Mischung 0,2 bis 0,5 Gewichtsteile, vorzugsweise etwa 0,36, Silberoxyd zugegeben, welches ein solches Wachstum von Bakterien verhindert.

Für die rißfreie Herstellung der Mikrofilterscheiben ist die Wahl der Formen und die Art der Trocknung besonders wichtig. Bevorzugt wird daher eine Form aus einem geringfügig feuchten Gips angewendet, in welche die fertige Mischung gefüllt wird. Gips ist ein Material, welche saugfähig für Wasser ist, wobei völlig trockener Gips der Mischung so schnell Wasser entziehen würde, daß es zu einer Rißbildung kommen könnte. Daher werden geringfügig feuchte Gipsformen bevorzugt.

Um Scheiben mit einer Öffnung in der Mitte herzustellen, muß die Form einen zentralen Kern aufweisen, damit diese Öffnung direkt ausgespart bleibt. Da auch in diesem Bereich eine Rißbildung besonders leicht auftritt, sollte der Kern aus einem elastischen und saugfähigen Material bestehen, welches Verformungen der Scheibe beim Trocknen nachgibt, jedoch als Form stabil genug ist. Besonders geeignet ist ein Hohlzylinder aus Papier oder Pappe, welcher zunächst auch Feuchtigkeit aufsaugt, sich beim späteren Trocknen geringfügig verformen kann und schließlich leicht abzulösen ist.

Beim Brennen einer so vorgefertigten und vorgetrockneten Scheibe entsteht im allgemeinen eine verdichtete Oberfläche, welche für den vorgesehenen Zweck, nämlich die Filterung von Flüssigkeiten, zu undurchlässig und daher ungeeignet ist. Die Oberfläche wird daher nach dem Brennen mit einem abtragenden Werkzeug von der verdichteten Deckschicht befreit, wobei diese im allgemeinen eine Dicke von etwa 5 µ hat.

Schließlich kann die Scheibe maßgenau geschnitten werden, was bevorzugt mit einem Hochdruckwasserstrahlschneider erfolgt. Um die Scheibe dichtend in eine Filtervorrichtung einsetzen zu können, werden der Außen- und Innenrand der Platte mit einer die Ränder zumindest auf einer Seite der Platte umgreifenden Dichtschicht versehen. Die Dichtschicht kann vorzugsweise aus einem Material auf Silikonbasis bestehen und an der Platte festgeklebt werden.

Im folgenden werden anhand der Zeichnung und anhand besonderer Ausführungsbeispiele, auf die die Erfindung jedoch nicht beschränkt ist, weitere Einzelheiten, Vorteile und Modifikationen der Erfindung beschrieben.
- Fig. 1: zeigt eine Ansicht von oben auf eine erfindungsgemäße Scheibe,
- Fig. 2: einen Querschnitt durch eine solche Scheibe und
- Fig. 3: eine zur Herstellung einer solchen Scheibe geeignete Form.

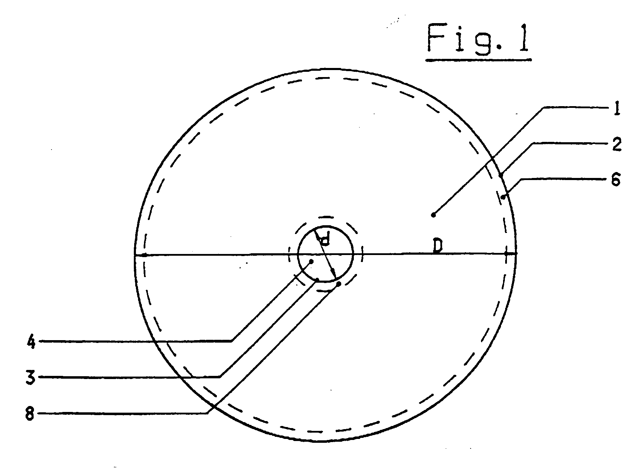

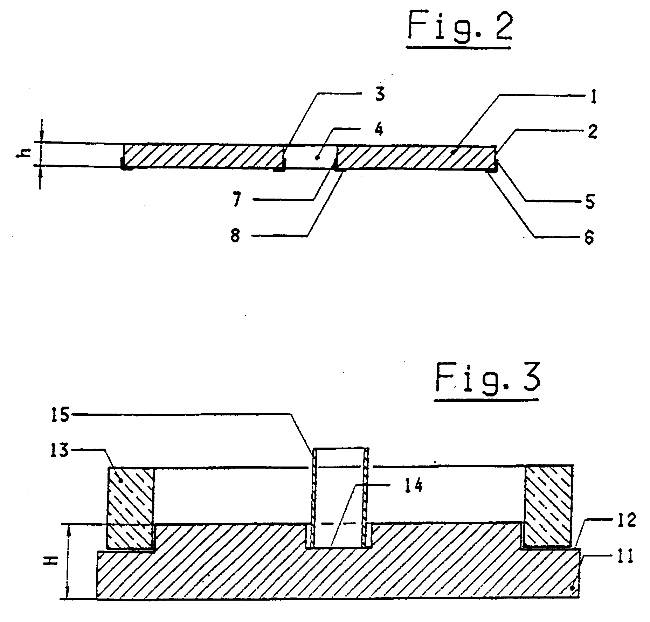

Die in den Figuren 1 und 2 dargestellte Ausführungsform einer Scheibe 1 hat einen Außendurchmesser D von etwa 40 cm und einen Innendurchmesser d der inneren Öffnung 4 von etwa 6,5 cm. Wie aus Figur 2 zu ersehen ist, ist der Außenrand 2 der Scheibe 1 mit einer Dichtung 5 versehen, welche mit einem Abschnitt 6 auch die Unterseite der Scheibe 1 umfaßt. Ebenso ist der Innenrand 3 der Scheibe 1 mit einer Dichtung 7 versehen, welche in einem Abschnitt 8 ebenfalls die Unterseite der Scheibe 1 umfaßt. Die Dichtabschnitte 6, 8 an der Unterseite der Scheibe 1 ermöglichen das abdichtende Auflegen auf eine die Scheibe 1 tragende Struktur, während die an dem Außenrand 2 und dem Innenrand 3 angebrachten Dichtungen 5, 6, zur Abdichtung gegenüber einer nicht dargestellten Behälterwand bzw. einer inneren zylindrischen Säule und zur Aufnahme geringfügiger Dehnungen der Scheibe unter Temperatureinfluß dienen. Die Dichtungen 5, 7 an dem Außen- bzw. Innenrand können auch getrennt von den Dichtungen 6, 8 an der einen Seite der Scheibe aufgebracht, vorzugsweise aufgeklebt, sein.

Die in Figur 3 schematisch dargestellte Form dient zur Herstellung einer erfindungsgemäßen Scheibe. Eine Grundplatte 11 mit einer Höhe H von etwa 50 bis 70 mm weist eine äußere Ringnut 12 auf, in welche ein ringförmiges Formteil 13 eingesetzt ist. Die Grundplatte 11 sollte eine Höhe H von 1/6 bis 1/10, vorzugsweise 1/8 des Durchmessers D der Scheibe 1 haben. Grundplatte 11 und ringförmiges Formteil 13 bestehen vorzugsweise aus Gips, welcher beim Füllen der Form nicht vollständig trocken sein soll. Die Grundplatte 11 weist außerdem eine kreis- oder ringförmige Vertiefung 14 in der Mitte auf, in welche ein hohlzylinderförmiger Kern 15, vorzugsweise aus Papier oder Pappe eingesetzt wird. In die durch Grundplatte 11, ringförmiges Formteil 13 und zylinderförmigen Einsatz 15 gebildete Form kann die zur Herstellung einer Scheibe 1 benötigte keramische Masse gegossen werden. Die Form saugt einen Teil der Flüssigkeit aus der abgegossenen Masse langsam auf und unterstützt so einen langsamen Trockungsprozeß. Um ein Verkleben der abgegossenen Masse mit der Gipsform zu vermeiden, kann diese mit einem Gleitmittel, insbesondere Talkum, bestrichen werden. Nach zwei bis fünf Stunden können die geformten Scheiben aus der Form genommen und auf einer glatten, saugfähigen Unterlage, vorzugsweise wiederum einer Gipsplatte, zum Trocknen ausgelegt werden. Um Risse zu vermeiden sollte die Trocknung bei Temperaturen zwischen 30 und 50 ° C, vorzugsweise 35 bis 40 ° C langsam erfolgen, so daß der Trocknungsverlauf etwa zwei bis drei Wochen dauern kann.

Nach der Trocknung erfolgt das Brennen der Scheibe, wobei bei einer Gesamtbranntdauer von 10 bis 15 Stunden eine Maximaltemperatur von etwa 1035 ° C für etwa 20 Minuten gehalten wird.

Im folgenden seien nochmals die Zutaten für die keramische Masse und das Verfahren bei ihrer Mischung anhand eines Ausführungsbeispiels, welches als besonders günstig angesehen wird, beschrieben.

In eine Vorlage von 4,4 l Wasser werden unter ständigem Rühren 6,5 g eines Verflüssigungsmittels, welches die Oberflächenspannung herabsetzt, und 12 g eines organischen Bindemittels zugegeben. Anschließend werden 900 g von einem ersten Kieselgur, 200 g von einem zweiten, vorkalzinierten Kieselgur, 460 g von einem dritten Kieselgur und 650 g Kaolin zugegeben. Nach intensivem Rühren werden außerdem 8 g Silberoxid beigemischt. Zur völligen Homogenisierung der gesamten Mischung wird diese ca. 15 bis 18 Stunden weitergerührt, wobei die Mischung abgußbereit ist, wenn sich die Viskosität fast sprunghaft um 10 bis 20 % verringert hat, d.h. die Mischung dünnflüssiger geworden ist. Der PH-Wert beim Ausgießen sollte bei ca. 9 liegen.

Die einzelnen Komponenten, insbesondere das Verflüssigungsmittel, das organische Bindemittel, können ihrerseits in Wasser gelöst zugegeben werden, wobei insgesamt für die Mischung eine Menge von 5 l Wasser erreicht werden soll.

Die folgende Tabelle enthält Angaben über die verwendeten unterschiedlichen Arten von Kieselgur bezüglich ihrer Zusammensetzung und ihrer Partikelgröße. Kieselgur, auch Diatomeerde genannt, ist ein natürlich vorkommendes Material, welches durch geologische Prozesse aus Kieselalgen entstanden ist. Solche Kieselgure sind unter dem Markennamen "Celite" von der Manville International Corporation, Denver, USA, erhältlich und werden mit den entsprechenden Zusammensetzung und sehr feinkörnig angeboten.

| Eigenschaft | Kieselgur 1 | Kieselgur 2 | Kieselgur 3 |
|---|---|---|---|
| Vorkalziniert | nein | ja | nein |
| Si 0₂ 0₂ | 85,8 | 85,8 | 89,3 |
| Al₂ 0₃ | 3,8 | 3,8 | 4,2 |
| Fe₂ 0₃ | 1,2 | 1,2 | 1,4 |
| Ti 0₂ | 0,2 | 0,2 | 0,2 |
| Ca 0 | 0,5 | 0,5 | 0,6 |
| Mg 0 | 0,6 | 0,6 | 0,6 |
| Na₂ 0 oder K₂ 0 | 1,1 | 1,1 | 3,5 |
| P₂ 0 | 0,2 | 0,2 | - |

Erfindungsgemäße Scheiben eignen sich in besonderer Weise aufgrund ihrer monolithischen flachen Form für selbstreinigende Filtereinrichtungen, wie sie in der europäischen Patentanmeldung 93 810 843.0 vom 03.11.1992 beschrieben werden. Auf diese Anmeldung wird zur Vermeidung von Wiederholungen vollinhaltlich bezug genommen. Gemäß der dort beschriebenen Anwendungsform werden zwei Filterscheiben durch einen Stützrahmen gegeneinander abgestützt als Filterelement verwendet, welches von außen mit ungereinigtem Wasser beaufschlagt wird, welches innen gereinigt wieder abgezogen werden kann. Viele solche Filterscheiben übereinandergestapelt bilden einen Filter mit großer Oberfläche und damit großem Durchfluß pro Zeiteinheit. Die flache Form der Scheiben ermöglicht die Reinigung durch rotierende Bürsten, welche an einer Mittelachse befestigt sind, die durch die innere Öffnung jeder Scheibe hindurchgeführt ist. Von den Scheiben werden bei jeder Reinigung dünne Oberflächenschichten abgetragen, wodurch sich die ursprünglichen Eigenschaften der frischen Filterplatte wiedereinstellen. Mittels Ultraschall oder anderer Überwachungstechniken kann festgestellt werden, wenn die Scheiben bis zu einer Dicke von beispielsweise etwa 0,5 cm abgetragen sind. In diesem Falle besteht bei weiterer Benutzung Bruchgefahr, so daß die Scheiben ausgetauscht werden müssen. Die erfindungsgemäßen Scheiben sind aufgrund ihrer monolithischen Gestalt auch einfach auszuwechseln und erfordern keine komplizierten Strukturen zu ihrer gegenseitigen Abstützung und Abdichtung. Sie eignen sich daher in besonderer Weise für einen wirtschaftlichen Betrieb von Filtereinrichtungen zur Entkeimung von Wasser. Die besonders kleinen Poren ermöglichen eine sichere mechanische Zurückhaltung aller Bakterien. Zur geschmacklichen Aufbereitung kann gegebenenfalls noch ein Aktivkohlefilter nachgeschaltet werden. Auch können übliche Entkeimungsmittel auf der "Reinseite" der Filterplatten zwischen diesen angeordnet werden.

## Patentansprüche

1. Mikrofilterscheibe (1) auf Kieselgurbasis, insbesondere zur Entkeimung von Wasser, mit folgenden Merkmalen:
a. die Mikrofilterscheibe (1) enthält Anteile von mindestens zwei silicatisch gebundenen Kieselgurarten, von denen mindestens eine vorkalziniert und später gebrannt ist;
b. die Mikrofilterscheibe (1) weist Porengrößen in der Größenordnung von 0.05 bis 0,5 µ, vorzugsweise 0,1 bis 0,3 µ, auf.

2. Mikrofilterscheibe (1) nach Anspruch 1, dadurch gekennzeichnet, daß die Mikrofilterscheibe (1) monolithisch und rund ist und einen Durchmesser (D) von mindestens 25 cm, vorzugsweise mindestens 35 cm, hat.

3. Mikrofilterscheibe (1) nach Anspruch 1 oder 2, dadurch gekennzeichnet, daß die Mikrofilterscheibe (1) eine Dicke (h) von 0,5 bis 2 cm, vorzugsweise etwa 1 bis 1,5 cm, aufweist.

4. Mikrofilterscheibe (1) nach einem der Ansprüche 1 bis 3, dadurch gekennzeichnet, daß die Mikrofilterscheibe (1) eine zentrale, vorzugsweise runde Öffnung (4) aufweist, mit einem Durchmesser (d) von 3 bis 15 cm, vorzugsweise 6 bis 8 cm.

5. Mikrofilterscheibe (1) nach einem der vorhergehenden Ansprüche, dadurch gekennzeichnet, daß die Außen- (2) und Innenränder (3) der Mikrofilterscheibe (1) maßhaltig geschnitten sind, vorzugsweise mit einem Hochdruckwasserstrahlschneider.

6. Mikrofilterscheibe (1) nach einem der vorhergehenden Ansprüche, dadurch gekennzeichnet, daß die Außen- (2) und Innenränder (3) der Mikrofilterscheibe (1) mit Dichtungen (5, 6, 7, 8) versehen sind, welche die Scheibe an einer Seite um 1 bis 8 mm, vorzugsweise 3 bis 5 mm umgreifen.

7. Mikrofilterscheibe (1) nach einem der vorhergehenden Ansprüche, dadurch gekennzeichnet, daß die Scheibe eine offenporige Oberfläche aufweist, welche so weich ist, daß mittels einer Reinigungsbürste mit Nylonborsten, Edelmetallborsten oder dergleichen ein Abrieb von 0,1 bis 0,5 µ in wenigen Arbeitsgängen erzielbar ist.

8. Mikrofilterscheibe (1) nach einem der vorhergehenden Ansprüche, dadurch gekennzeichnet, daß die Scheibe nicht auswaschbare Anteile an Silbersalzen enthält.

9. Mikrofilterscheibe (1) nach einem der vorhergehenden Ansprüche, dadurch gekennzeichnet, daß die Scheibe mindestens drei Kieselgurkomponenten und einen Anteil Kaolin enthält.

10. Mikrofilterscheibe (1) nach einem der vorhergehenden Ansprüche, dadurch gekennzeichnet, daß die Scheibe etwa 6 bis 12 Gewichtsprozent (bezogen auf die trockene Scheibe), vorzugsweise etwa 9 Gewichtsprozent, vorkalzinierten und gemeinsam mit den übrigen Bestandteilen nochmals gebrannten Kieselgur, 25 bis 30 Gewichtsprozent Kaolin und als Rest mindestens eine weitere Sorte Kieselgur enthält und gegebenenfalls Silbersalze und herstellungsbedingte geringe andere Bestandteile.

11. Verfahren zur Herstellung einer Mikrofilterscheibe (1) mit Porengrößen von 0.05 bis 0,5 µ, vorzugsweise 0,1 bis 0,3 µ, und mit einem Durchmesser (D) größer als 25 cm, vorzugsweise größer als 35 cm, insbesondere einer Filterscheibe nach einem der Ansprüche 1 bis 10, mit folgenden Schritten:
a. es werden gemischt: mindestens 6 bis 12 Gewichtsteile, vorzugsweise etwa 9, einer schon kalzinierten Sorte Kieselgur mit 25 bis 35 Gewichtsteilen, vorzugsweise etwa 30, Kaolin und 53 bis 69 Gewichtsteile, vorzugsweise etwa 61, mindestens einer nicht oder nur wenig kalzinierten Sorte Kieselgur;
b. die Mischung wird angerührt in etwa 200 bis 250 Gewichtsteilen, vorzugsweise etwa 225, Wasser unter Zugabe von 0,2 bis 0,35 Gewichtsteilen, vorzugsweise 0,27, mindestens eines Verflüssigungsmittels und von 0,4 bis 0,8 Gewichtsteilen, vorzugsweise 0,5 bis 0,7, eines organischen Bindemittels;
c. die Mischung wird mindestens so lange gerührt, bis sich eine deutliche Viskositätsänderung einstellt, vorzugsweise etwa 12 bis 15 Stunden;
d. die fertiggerührte Mischung wird in eine Form (11, 13, 15) aus feuchtigkeitsaufnehmendem Material, vorzugsweise Gips, gegossen;
e. die Mischung wird langsam getrocknet, vorzugsweise über einen Zeitraum von 8 bis 25 Tagen, insbesondere 10 bis 20 Tagen;
f. die getrocknete Mischung wird gebrannt, vorzugsweise bei einer Maximaltemperatur von 1000 bis 1050 °C, insbesondere 1035 °C.

12. Verfahren nach Anspruch 11, dadurch gekennzeichnet, daß folgende Gewichtsteile verwendet werden:
etwa 40,72 eines ersten unkalzinierten oder wenig kalzinierten Kieselgurs,
etwa 9.05 eines zweiten kalzinierten Kieselgurs,
etwa 20,82 eines dritten unkalzinierten oder wenig kalzinierten Kieselgurs und
etwa 29,41 Kaolin.

13. Verfahren nach einem der Ansprüche 11 oder 12, dadurch gekennzeichnet, daß der Mischung 0,2 bis 0,5 Gewichtsteile, vorzugsweise etwa 0,36, Silberoxid zugegeben werden.

14. Verfahren nach einem der Ansprüche 11, 12 oder 13, dadurch gekennzeichnet, daß die Form (11, 13) aus einem geringfügig feuchten Gips besteht.

15. Verfahren nach einem der Ansprüche 11 bis 14, dadurch gekennzeichnet, daß die Form (11, 13, 15) in der Mitte (14) mit einem zentralen Kern (15) aus einem elastischen und saugfähigen Material versehen wird, vorzugsweise einem Hohlzylinder aus Papier oder Pappe.

16. Verfahren nach einem der Ansprüche 11 bis 15, dadurch gekennzeichnet, daß nach dem Brennen die Oberfläche der Scheibe mit einem abtragenden Werkzeug von einer verdichteten Deckschicht mit einer Dicke von etwa 5 µ befreit wird.

17. Verfahren nach einem der Ansprüche 11 bis 16, dadurch gekennzeichnet, daß die gebrannte Mikrofilterscheibe (1) mit einem Hochdruckwasserstrahlschneider maßgenau geschnitten wird.

18. Verfahren nach einem der Ansprüche 11 bis 17, dadurch gekennzeichnet, daß der Außen- und Innenrand der Platte mit die Ränder zumindest auf einer Seite umgreifenden Dichtungen (5, 6, 7, 8) versehen werden, vorzugsweise auf Silikonbasis.
